# EUROPÄISCHE PATENTANMELDUNG

(11) **EP 4 194 432 A1**
(43) Veröffentlichungstag der Anmeldung: **14.06.2023**
(21) Anmeldenummer: 21020615.7
(22) Anmeldetag: 07.12.2021
(51) Int. Cl.: C07C 41/01, C07C 43/04, C07C 29/152, C07C 31/04, C07C 41/09, B01J 8/04

(54) **REAKTOR UND VERFAHREN ZUR HERSTELLUNG VON METHANOLFOLGEPRODUKTEN UND INSBESONDERE DIMETHYLETHER**

(71) Anmelder: Linde GmbH, 82049 Pullach (DE)
(72) Erfinder: Schödel, Nicole, 82049 Pullach (DE); Winkler, Florian, 82049 Pullach (DE); Behrens, Axel, 82049 Pullach (DE); Peschel, Andreas, 82049 Pullach (DE); Klendl, Isabel, 80799 München (DE)
(74) Vertreter: Fischer, Werner

(57) **Zusammenfassung**

Die Erfindung betrifft einen Reaktor (200, 300, 400) zur katalytischen Herstellung von Methanolfolgeprodukten, insbesondere Dimethylether, aus Synthesegas, der als Rohrbündelreaktor mit einem aus Rohren (140) gebildeten Rohrbündel und zumindest einem, die Rohre umgebenden Mantel, der einen eingangsseitigen und einen ausgangsseitigen Rohrboden aufweist, wobei die Rohrböden einen Gasraum, der die Rohre (140) umfasst, von einem Kühlmedienraum trennen, ausgestaltet ist und zumindest eine erste (110) und eine zweite (120) Reaktionszone aufweist, wobei die erste Reaktionszone (110) mit einem ersten Katalysatorbett beaufschlagt ist und die zweite Reaktionszone (120) mit einem zweiten Katalysatorbett beaufschlagt ist, dadurch gekennzeichnet, dass die zweite Reaktionszone (120) bei Betrieb des Reaktors (200, 300, 400) stromab der ersten Reaktionszone (110) angeordnet ist, in beiden Reaktionszonen (110, 120) das Katalysatorbett dazu eingerichtet ist die Direktsynthese der Methanolfolgeprodukte zu katalysieren, das erste Katalysatorbett dazu eingerichtet ist, eine Reaktion von Wasserstoff und Kohlenstoffmonoxid und/oder Kohlenstoffdioxid zu Methanol durch dessen höheren verhältnismäßigen Anteil an katalytisch aktivem Material gegenüber der zweiten Reaktionszone zu favorisieren, und der Wärmeübergang von der ersten Reaktionszone (110) auf ein die Reaktionszone umgebendes Kühlmedium den Wärmeübergang von der zweiten Reaktionszone (120) auf das Kühlmedium übersteigt. Ferner wird ein Verfahren vorgeschlagen, in dem ein solcher Reaktor verwendet wird.

## Beschreibung

Die Erfindung betrifft einen Reaktor und ein Verfahren zur Herstellung von Methanolfolgeprodukten und insbesondere Dimethylether aus Synthesegas.

### Hintergrund der Erfindung

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether kann entweder direkt aus Methanol oder indirekt aus Erd- oder Biogas erzeugt werden. Im letzteren Fall wird das Erd- oder Biogas zunächst zu Synthesegas reformiert. Synthesegas ist ein Gasgemisch, das zumindest Kohlenstoffmonoxid und Wasserstoff in wechselnden Anteilen, die zusammengenommen jedoch den überwiegenden Teil des Gasgemischs ausmachen, enthält. Synthesegas kann auch mittels anderer Verfahren, beispielsweise durch Pyrolyse von Kohle, Öl, kohlenstoffhaltigen Abfällen, Biomasse oder anderen kohlenstoffhaltigen Ausgangsstoffen, durch Trockenreformierung (DryReforming) von Erdgas mit Kohlenstoffdioxid, durch Dampfreformierung (Steam Reforming) von Erdgas, durch autotherme Reformierung (ATR) von Erdgas, durch partielle Oxidation (POX) von Kohlenwasserstoffen, insbesondere Erdgas bzw. Methan, oder Kombinationen aus den genannten Verfahren, gewonnen werden. Das Synthesegas wird anschließend entweder in einer zweistufigen Reaktion zu Methanol und anschließend zu Dimethylether oder in einer einstufigen Reaktion direkt zu Dimethylether konvertiert.

Die Synthese von Dimethylether aus Synthesegas ist thermodynamisch und wirtschaftlich vorteilhaft gegenüber einer Synthese aus Methanol.

Unter Synthesegas (kurz Syngas) wird im Rahmen dieser Erfindung jedes Fluid verstanden, das zu einem überwiegenden Anteil aus Wasserstoff und Kohlenstoffmonoxid gebildet ist, unabhängig von der Herkunft eines solchen Fluides. Synthesegas im Sinne dieser Erfindung kann auch nicht unerhebliche Anteile an Kohlenstoffdioxid aufweisen.

Die vorliegende Erfindung betrifft insbesondere die einstufige Synthese von Dimethylether, wobei unter einer "einstufigen" Synthese eine Synthese verstanden wird, bei der sämtliche Reaktionen in einunddemselben Reaktor und in einunddemselben Katalysatorbett ablaufen. Nachfolgend wird für eine einstufige Synthese von Dimethylether auch der Begriff "Direktsynthese" verwendet. Bei den ablaufenden Reaktionen handelt es sich um eine Reaktion von Wasserstoff mit Kohlenstoffmonoxid und/oder Kohlenstoffdioxid zu Methanol und eine (Weiter-) Reaktion von Methanol zu Dimethylether und Wasser. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300°C und bei einem Druck von 20 bis 100 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor erhaltener Produktstrom wird kopfseitig entnommen, gekühlt und einer Trennung zugeführt.

Der Produktstrom enthält typischerweise neben Dimethylether nicht umgesetzte Komponenten des Synthesegases sowie weitere Reaktionsprodukte. Typischerweise weist der Produktstrom neben Dimethylether zumindest Methanol, Wasser, Kohlenstoffdioxid, Kohlenstoffmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlenstoffdioxid und leichter als Kohlenstoffdioxid siedende Komponenten wie Wasserstoff und Kohlenstoffmonoxid enthalten. Zur spezifikationsgerechten Gewinnung von Dimethylether müssen diese zumindest teilweise abgetrennt werden.

Die Zusammensetzung des Produktstroms wird dabei insbesondere durch die gewählten Reaktionsbedingungen während der Synthese bestimmt. Je näher diese stromab des Reaktors an der geforderten Spezifikation liegt, desto geringer ist der Aufwand, der zur Aufreinigung des Produktstroms betrieben werden muss.

Es soll daher ein verbessertes Reaktorkonzept für die einstufige Synthese von Dimethylether aus Synthesegas angegeben werden.

### Offenbarung der Erfindung

Diese Aufgabe wird durch einen Reaktor zur Herstellung von Methanolfolgeprodukten, insbesondere Dimethylether, und ein Verfahren, das einen solchen Reaktor verwendet, mit den Merkmalen der jeweiligen unabhängigen Patentansprüche gelöst. Vorteilhafte Weiterbildungen der Erfindung sind Gegenstand der abhängigen Patentansprüche sowie der folgenden Beschreibung.

Vor der Erläuterung der Merkmale und Vorteile der vorliegenden Erfindung werden deren Grundlagen und die verwendeten Begriffe erläutert.

Ein Fluid (die Bezeichnung "Fluid" wird nachfolgend auch kurz für entsprechende Ströme, Fraktionen usw. verwendet) ist von einem anderen Fluid (das auch als Ausgangsfluid bezeichnet wird) "abgeleitet" oder aus einem solchen Fluid "gebildet", wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Strom kann auch beispielsweise einfach dadurch "gebildet" werden, dass er aus einem Speicherbehälter abgezogen wird.

Fluide können im hier verwendeten Sprachgebrauch reich oder arm an einer oder mehreren enthaltenen Komponenten sein, wobei "reich" für einen Gehalt von wenigstens 50%, 75%, 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Sie können im hier verwendeten Sprachgebrauch angereichert oder abgereichert an einer oder mehreren Komponenten sein, wobei sich diese Begriffe auf einen entsprechenden Gehalt in einem Ausgangsfluid beziehen, aus dem das Fluid gebildet wurde. Das Fluid ist "angereichert", wenn es zumindest den 1,1-fachen, 1,5-fachen, 2-fachen, 5-fachen, 10-fachen 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsfluid, enthält. Ein "überwiegend" eine oder mehrere Komponenten enthaltendes Fluid enthält diese eine oder mehreren Komponenten zu wenigstens 50%, 75%, 90%, 95%, 98% oder 99% bzw. ist reich an diesen.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte verwendet werden müssen, um ein erfinderisches Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Unterschiedliche Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Ist nachfolgend kurz von einer "Synthese" von Methanolfolgeprodukten, insbesondere von Dimethylether, die Rede, wird hierunter ein Verfahren verstanden, bei dem ein Synthesegas bzw. Syngas enthaltender Einsatz, also ein Gasgemisch, das in geeigneten Anteilen zumindest Kohlenstoffmonoxid und Wasserstoff enthält, zu einem entsprechenden Methanolfolgeprodukte, beispielsweise Dimethylether, enthaltenden Produktstrom umgesetzt wird. Der Anteil an Wasserstoff in dem Synthesegas kann dabei beispielsweise unter Verwendung einer Wassergas-Shift-Reaktion erhöht werden. Dabei reagiert in dem Synthesegas vorhandenes Kohlenstoffmonoxid mit zu diesem Zweck zugegebenem Wasser(dampf) zu Kohlenstoffdioxid, das gegebenenfalls abgetrennt werden kann. Ein derart nachbehandeltes Synthesegas wird auch als geshiftetes Synthesegas bezeichnet.

Generell sollen hier unter einer "Wassergasshift" immer beide Richtungen der mit diesem Begriff bezeichneten Gleichgewichtsreaktion verstanden werden, also die Reaktion von Kohlenmonoxid mit Wasser zu Kohlendioxid und Wasserstoff und umgekehrt (letztere auch als "reverse" Wassergasshift, RWGS, bezeichnet). Die Richtung der Wassergasshift wird dem Bedarf entsprechend eingesetzt.

Ein entsprechender Produktstrom der Synthese von Methanolfolgeprodukten, insbesondere Dimethylether, enthält aufgrund der nicht vollständigen Reaktion und aufgrund des Auftretens von Nebenreaktionen bei der Synthese, insbesondere in Abhängigkeit von den Charakteristika der verwendeten Katalysatoren und den jeweiligen Gehalten der Komponenten des Synthesegases, nicht ausschließlich das jeweilige Methanolfolgeprodukt, beispielsweise Dimethylether, sondern weitere Verbindungen. Diese sind zumindest Methanol, Wasser, Kohlenstoffdioxid, Kohlenstoffmonoxid und Wasserstoff, jedoch typischerweise auch geringere Mengen Methan, Ethan, organische Säuren und höhere Alkohole. Diese genannten weiteren Verbindungen müssen, wie erwähnt, abgetrennt werden. Die Abtrennung erfolgt einerseits, um nachfolgende Trennschritte zu ermöglichen, und andererseits, um das Methanolfolgeprodukt, z.B. Dimethylether, in der geforderten Reinheit, also "spezifikationsgerecht" zu gewinnen. Wie ebenfalls bereits erwähnt, ist diese Abtrennung umso unproblematischer, je reiner der Produktstrom die Reaktion verlässt.

### Merkmale und Vorteile der Erfindung

Im Einzelnen schlägt die Erfindung einen Reaktor zur katalytischen Herstellung von Methanolfolgeprodukten, insbesondere Dimethylether, aus Synthesegas vor, der als Rohrbündelreaktor ausgestaltet ist und zumindest eine erste und eine zweite Reaktionszone aufweist, wobei die erste Reaktionszone mit einem ersten Katalysatorbett beaufschlagt ist und die zweite Reaktionszone mit einem zweiten Katalysatorbett beaufschlagt ist. Der Reaktor ist dabei dadurch gekennzeichnet, dass die zweite Reaktionszone bei Betrieb des Reaktors stromab der ersten Reaktionszone angeordnet ist.

Der Reaktor weist ein Rohrbündel auf, das eine erste Gruppe von Rohren, nachfolgend als "erstes Rohrteilbündel" bezeichnet, und eine zweite Gruppe von Rohren, nachfolgend als "zweites Rohrteilbündel" bezeichnet, umfasst, wobei die Rohre des ersten Rohrteilbündels insbesondere zumindest teilweise parallel neben den Rohren des zweiten Rohrteilbündels verlaufen, und wobei die erste Reaktionszone in den Rohren des ersten Rohrteilbündels ausgebildet ist und die zweite Reaktionszone in den Rohren des zweiten Rohrteilbündels ausgebildet ist. Die Rohre des ersten Rohrteilbündels und die Rohre des zweiten Rohrteilbündels sind insbesondere in einem gemeinsamen, aber ggf. längs der Rohre unterteilten Mantelraum angeordnet.

Es ist, mit anderen Worten, insbesondere ein Rohrbündel vorgesehen, wobei die Rohre der Rohrteilbündel gaseintrittsseitig, und in einer speziellen Ausgestaltung auch gasaustrittsseitig, voneinander getrennt sind. Eine Trennung liegt dann vor, wenn die Rohrteilbündel nicht ausgehend von einem gemeinsamen Gasraum innerhalb des Mantelraums verlaufen bzw. nicht in einen gemeinsamen Gasraum innerhalb des Mantelraums münden, beispielsweise über entsprechende Rohrböden. Der Begriff des Mantelraums bezeichnet hier einen insbesondere zylindrischen Behälter, innerhalb dessen das Rohrbündel verläuft.

In einer weiteren Ausführungsform ist das erstes Rohrteilbündel als ein Rohrbündel gestaltet und das zweite Rohrteilbündel als ein weiteres Rohrbündel gestaltet, wobei die Rohre des ersten Rohrteilbündels zumindest teilweise parallel und fluchtend mit den Rohren des zweiten Rohrteilbündels verlaufen, und wobei die erste Reaktionszone in den Rohren des ersten Rohrteilbündels ausgebildet ist und die zweite Reaktionszone in den Rohren des zweiten Rohrteilbündels ausgebildet ist. Unter einer "fluchtenenden" Anordnung sei dabei insbesondere eine Anordnung hintereinander verstanden, bei der die Mittelachsen der Rohre auf einer gemeinsamen Linie liegen. Die Rohre des ersten Rohrteilbündels und die Rohre des zweiten Rohrteilbündels sind in einem gemeinsamen Mantelraum angeordnet.

In einer speziellen Ausführungsform ist das erstes Rohrteilbündel als ein Rohrbündel gestaltet, das zweite Rohrteilbündel als ein zweites Rohrbündel gestaltet, wobei die Rohre des ersten Rohrteilbündels in einem ersten Mantelraum angeordnet sind und die Rohre des zweiten Rohrteilbündels in einem zweiten Mantelraum ausgebildet sind. Bevorzugt ist jedoch die Anordnung in einem gemeinsamen Mantelraum und besonders bevorzugt die Anordnung in einem Mantelraum und nebeneinander liegenden (anti)parallel verlaufenden Reaktionszonen.

Die beiden Reaktionszonen sind für eine einstufige Synthese bzw. Direktsynthese von Methanolfolgeprodukten, z.B. Dimethylether, im oben erläuterten Sinn ausgestaltet, also eine Reaktion von Wasserstoff und Kohlenstoffmonoxid und/oder Kohlenstoffdioxid zu Methanol und eine Reaktion von Methanol zu einem oder mehreren Methanolfolgeprodukten und Wasser. Das erste Katalysatorbett ist dabei dazu eingerichtet ist, überwiegend die Reaktion von Wasserstoff mit Kohlenstoffmonoxid und/oder Kohlenstoffdioxid zu Methanol (aber ggf. zu einem geringeren Anteil auch die Reaktion von Methanol zu dem bzw. den Methanolfolgeprodukt(en) und Wasser) zu katalysieren, und das zweite Katalysatorbett ist dazu eingerichtet ist, überwiegend eine Reaktion von Methanol zu dem bzw. den Methanolfolgeprodukt(en) und Wasser (aber ggf. zu einem geringeren Anteil auch die Reaktion von Wasserstoff und Kohlenstoffmonoxid bzw. Kohlenstoffdioxid zu Methanol) zu katalysieren. Die Unterschiede ergeben sich insbesondere durch einen höheren verhältnismäßigen Anteil an katalytisch aktivem Material für die die Reaktion von Wasserstoff mit Kohlenstoffmonoxid und/oder Kohlenstoffdioxid zu Methanol in der ersten gegenüber der zweiten Reaktionszone. Die zweite Reaktionszone befindet sich im Betrieb des Reaktors stromab der ersten.

Die einzelnen Rohrteilbündel, in denen die zumindest zwei Reaktionszonen angeordnet sind, werden erfindungsgemäß hinsichtlich der jeweiligen örtlich ablaufenden Hauptreaktion und der daraus resultierenden unterschiedlichen benötigten Wärmeabfuhr sowie katalytischen Anforderungen angepasst und optimiert bzw. ausgestaltet. Dabei ist die erste Reaktionszone so gestaltet, dass sich darin beim Betrieb des Reaktors ein höherer volumenbezogener Wärmeübergang von der Reaktionszone auf ein die Reaktionszone umgebendes Kühlmedium einstellt, als dies in der zweiten Reaktionszone der Fall ist. Die erste Reaktionszone ist dabei erfindungsgemäß für einen Betrieb mit einem ersten volumenbezogenen Wärmeübergang (bzw. Wärmeübertragungskapazität, die z.B. von Strömungsgeschwindigkeiten der beteiligten Fluide, deren Wärmekapazitäten, Rohrwandstärken, Wärmeleitfähigkeit des Rohrmaterials, Verhältnis von Rohroberfläche zu Rohrvolumen und Temperaturunterschied über die Rohrwand abhängig ist) und die zweite Reaktionszone für einen Betrieb mit einem zweiten volumenbezogenen Wärmeübergang ausgebildet, wobei der erste Wärmeübergang den zweiten Wärmeübergang übersteigt.

Beispielweise kann ein höherer Wärmeübergang in der ersten Reaktionszone durch höhere Raumgeschwindigkeiten und/oder Lineargeschwindigkeiten innerhalb der Rohre erzielt werden. Dies kann bei gleichen Rohrdurchmessern und Rohrlängen der zwei Rohrteilbündel durch ein kleineres Volumen (beispielsweise durch geringere Rohranzahl) der ersten Reaktionszone gegenüber der zweiten Reaktionszone erreicht werden. Alternativ kann die Wärmetauscherfläche und damit die Wärmeabfuhr durch kleinere Innendurchmesser der Rohre der ersten Reaktionszone gegenüber der zweiten Reaktionszone bei gleichem Volumen und Länge der beiden Reaktionszonen erhöht werden.

Des Weiteren besteht die Möglichkeit einer Beeinflussung des äußeren Wärmeüberganges. So kann der Mantelraum so gestaltet sein, dass sich die Anströmrichtung des Kühlmediums und/oder die Anströmgeschwindigkeit des Kühlmediums an die Rohre der ersten Reaktionszone von der zweiten Reaktionszone unterscheiden. Dies kann z.B. durch Einbauten im Mantelraum realisiert werden. Durch Einbringen von Lenkblechen, können Bereiche mit quer angeströmten Rohren entstehen und Bereiche mit längsangeströmten Rohren entstehen, ersteres erhöht den Wärmeübergang. Die Anzahl der Lenkbleche erhöht die Anströmgeschwindigkeit und somit auch den äußeren Wärmeübergang.

Auch eine Kombination aus zwei oder mehr der genannten Möglichkeiten zur Steuerung des volumenbezogenen Wärmeübergangs ist gegebenenfalls vorteilhaft.

Durch die erfindungsgemäß vorgeschlagenen Maßnahmen können die Einzelreaktionen, die in der einstufigen Synthese bzw. Direktsynthese von Methanolfolgeprodukten wie Dimethylether in großer räumlicher Nähe zueinander, d.h. in demselben Katalysatorbett, ablaufen, jedoch je nach Fortschreiten der Reaktion unterschiedlich stark vertreten sind, ähnlich einer zweistufigen Synthese zumindest teilweise separat voneinander gesteuert werden. So werden die Vorteile der beiden Methoden miteinander verbunden. Insbesondere kann die Reaktion in einem einzigen Reaktor durchgeführt werden, ohne auf die gezielte Beeinflussung der Einzelreaktionen verzichten zu müssen bzw. eine Reaktion der anderen zu bevorzugen.

Dadurch, dass die erste Reaktionszone in einem ersten Rohrteilbündel und die zweite Reaktionszone in einem von dem ersten Rohrteilbündel separaten zweiten Rohrteilbündel angeordnet sind, werden klar definierte Katalysatorbetten für die jeweiligen Reaktionszonen mit entsprechend präziser und effizienter Reaktionsführung geschaffen.

Insbesondere ist die in der ersten Reaktionszone vorherrschende Reaktion von Synthesegas zu Methanol stärker exotherm als die Reaktion von Methanol zu dem bzw. den Methanolfolgeprodukten, insbesondere zu Dimethylether, so dass in der ersten Reaktionszone eine effizientere Wärmeabfuhr als in der zweiten Reaktionszone erforderlich sein kann. So kann das Volumen der ersten Reaktionszone gegenüber der zweiten bei gleichem Rohrinnendurchmesser und Rohrlänge geringer gewählt werden, um die Lineargeschwindigkeit entsprechend zu steuern. Oder bei gleichem Volumen weisen die Rohre des ersten Rohrteilbündels einen Durchmesser auf, der von einem Durchmesser von Rohren des zweiten Rohrteilbündels abweicht, um die Wärmetauscherfläche entsprechend anzupassen. Daher können die Rohre des ersten Rohrteilbündels mit kleinerem Durchmesser als die Rohre des zweiten Rohrteilbündels vorgesehen sein. Dies bietet den Vorteil, dass die unterschiedlichen Wärmetönungen der Teilreaktionen bereits beim Reaktordesign berücksichtigt werden. Auf diese Weise ergeben sich auch insbesondere die unterschiedlichen Wärmeübergänge, die erfindungsgemäß vorgesehen sind.

Dieser Umstand ermöglicht es, einen hohen Anteil an katalytisch aktivem Material für die Reaktion von Wasserstoff mit Kohlenstoffmonoxid und/oder Kohlenstoffdioxid zu Methanol in die erste Reaktionszone einzubringen, und diese somit optimal hinsichtlich ihrer Ausnutzung und Widerstandsfähigkeit gegen Katalysatorgifte und Alterungseffekte zu gestalten. Dadurch herrschen hier ähnliche Bedingungen wie bei konventionellen Methanolsyntheseprozessen, wobei im Rahmen der vorliegenden Erfindung durch Einbringen von Mindestmengen an katalytisch aktivem Material für die die Reaktion von Methanol zu dem bzw. den Methanolfolgeprodukten und Wasser die Ausnutzung durch höhere Gleichgewichtsumsätze zusätzlich optimiert werden kann.

Für die in der zweiten Reaktionszone hauptsächlich ablaufende Dehydratisierung wird, wie bereits erwähnt, eine wesentlich geringere Wärmeabfuhr zur Gewährleistung einer ausreichenden Kühlung benötigt. Zusätzlich ist das Edukt der in der zweiten Reaktionszone bevorzugt katalysierten Dehydratisierungsreaktion durch die hohen Synthesegasmengen verdünnt. Für eine hohe Konversion und Ausnutzung des Katalysators ist daher die erfindungsgemäße Reduktion der Raumgeschwindigkeit und/oder Lineargeschwindigkeit von Vorteil. Auch eine anderweitige Verringerung der Lineargeschwindigkeit und/oder Raumgeschwindigkeit in der zweiten Reaktionszone, beispielsweise durch eine geeignete Verringerung der in diese eingeleiteten Fluidmenge, ist in bestimmten Ausgestaltungen vorteilhaft.

Vorzugsweise werden die Innendurchmesser dementsprechend gewählt, so dass das Volumen der ersten Reaktionszone dem Volumen der zweiten Reaktionszone gleicht, dies ermöglicht ein symetrisches Reaktordesign und erleichtert das Einbringen von Einbauten in den Mantelraum. Werden die Rohre der beiden Rohrteilbündel fluchtend in einen Mantelraum angeordnet, so können die Reaktionszonen auch in ihrer jeweiligen Länge Unterschiede aufweisen. In einem Solchen Fall ist es vorteilhaft, die Rohrinnendurchmesser so zu wählen, dass die Querschnittsfläche der beiden Rohrteilbündel sich gleicht. Es können aber auch unterschiedliche Volumen der Reaktionszonen und Innendurchmesser der Rohre vorliegen.

Zur wesentlichen Unterstützung der Wärmeabfuhr im ersten Rohrteilbündel sind hohe Raumgeschwindigkeiten bzw. durch entsprechende Wahl der Rohrinnendurchmesser daraus resultierende Lineargeschwindigkeiten nötig. Die Raumgeschwindigkeiten für eine Methanolsynthese aus Synthesegas liegen typischerweise in einem Bereich von 5000-15000 h⁻¹, während die der Direktsynthese von Dimethylether in einem Bereich von 2000-4000 h-1 liegen. Dies zeigt bereits, dass bei gleich ausgestalteten Reaktionszonen in der Direktsynthese für eine ausreichende Wärmeabfuhr entweder der Anteil an aktivem Material für die Reaktion von Wasserstoff und Kohlenstoffmonoxid und/oder Kohlenstoffdioxid zu Methanol stark reduziert vorliegen muss, oder, um einen höheren Anteil einbringen zu können, der Rohrdurchmesser derart klein gewählt sein muss, dass ein ausreichender Wärmeübergang gewährleistet ist. Ersteres macht das Katalysatorbett stark anfällig und abhängig von den Alterungs-und Vergiftungserscheinungen, die typischerweise in der Methanolsynthese auftreten, wodurch auf Dauer die Dehydratisierung bevorzugt wird, letzteres führt zu einer erhöhten Anzahl an Rohren bis hin zu Baubarkeitsproblemen und zu einer Bevorzugung der Methanolsynthese.

Die Aufteilung der Reaktionszone für die Direktsynthese in zwei definierte Reaktionszonen entkoppelt sozusagen die Anforderungen der im vorderen Teil maßgeblichen Reaktion von Wasserstoff, Kohlenstoffmonoxid und/oder Kohlenstoffdioxid zu Methanol von den Anforderungen der Direktsynthese und ermöglicht somit einen kompakten und effizienten Reaktor.

Der Innendurchmesser der Rohre des ersten Rohrteilbündels kann beispielsweise aus einem Bereich von 20mm bis 60mm und der Innendurchmesser der Rohre des zweiten Rohrteilbündels aus einem Bereich von 20mm bis 80mm ausgewählt sein, wobei das Gesamtvolumen des ersten Rohrteilbündels vorteilhafterweise das Gesamtvolumen des zweiten Rohrteilbündels nicht überschreitet.

Das Verhältnis der Volumina bei gleichen Rohrdurchmessern und Rohrlängen der ersten zur zweiten Reaktionszone liegt dabei vorteilhafterweise zwischen 0,3:1 und 0,75:1 was gleichzeitig auch vorteilhafterweise umgekehrt für die jeweils herrschenden Raumgeschwindigkeiten gilt. Vorteilhafterweise weisen das erste und zweite Katalysatorbett Katalysatoren bzw. ein katalytisch aktives Material auf, das bzw. die einen oder mehrere Methanolsyntheskatalysatoren der Familie der Cu/ZnO/Al₂O3 oder Indium-basierte Systeme oder temperaturstabilere Varianten, wie z.B. auf Basis von Kupfer und/oder Zink mit Zirkonbeimengungen und oder Siliziumoxid, sowie Dehydratisierungskatalysatoren (z.B. gamma-Al₂O3, Aluminiumsilikate Al₂O₃/SiO₂, Zeolithe, SAPO-34, ZSM-5) und/oder bifunktionale Katalysatoren enthält. Hierbei handelt es sich jeweils besonders effiziente bzw. gut zu kontrollierende Katalysatorsysteme für die Synthese von Methanol aus Synthesegas bzw. die Synthese von Dimethylether bzw. anderen Methanolfolgeprodukten aus Methanol.

Unter anderem können die Dehydratisierungskatalysatoren auch Dehydratisierungen bis hin zu Olefinen bzw. im Speziellen Propylen und/oder Ethylen durchführen. Dies hängt von den Reaktionsbedingungen und im Speziellen von der Temperatur ab. Somit sind auch weitere Methanolfolgeprodukte unter Verwendung der genannten Katalysatorfamilien zugänglich. Unter Methanolfolgeprodukten sind unter anderem Ethylene, Propylene und höhere Olefine zu verstehen. In einer Direktsynthese von Methanolfolgeprodukten aus Synthesegas soll auch der höhere Gleichgewichtsumsatz ausgenutzt werden, wobei die gleiche Problematik der Eduktverarmung, Exothermiekontrolle und Methanolsynthesekatalysatorstabilität besteht. So kann auch für diese Reaktionen das beschriebene Reaktorkonzept mit unterschiedlichen Methanolsyntheskatalysatoranteilen in zwei unterschiedlichen Reaktionszonen vorteilhaft sein. Es kann in der ersten Reaktionszone auch eine andere Direktsynthese als in der zweiten Reaktionszone vorliegen, so dass z.B. in der ersten Reaktionszone eine Dimethyletherdirektsynthese stattfindet, die möglichst hohe Konversionen erreicht und in der zweiten Reaktionszone eine weitere Direktsynthese stattfindet die ein Olefin hervorbringt und nahe an den Gleichgewichtsumsatz führt. Dies ist möglich, da bekannte Prozesse wie Methanol zu Olefinen oder Methanol zu Propylen auch Dimethylether als Edukt benutzen können. Vorzugsweise sind dann die Reaktionszonen räumlich voneinander getrennt und/oder benutzen unterschiedliche Kühlkreisläufe mit unterschiedlichen Kühlmedien und/oder benutzen unterschiedliche, der jeweiligen Reaktion angepasste Methanolsynthesekatalysatoren und Dehydratisierungskatalysatoren der jeweiligen Reaktion.

Der besseren Lesbarkeit halber wird im Folgenden primär die Direktsynthese von Dimethylether als stellvertretendes Beispiel für Synthesen von Methanolfolgeprodukten beschrieben, ohne weitere bzw. andere Produkte auszuschließen.

Das Katalysatorbett kann als Mischung vorliegen oder als bifunktionaler Katalysator. Für ein besseres Verständnis wird hier im Weiteren von massenbezogenen Anteilen der unterschiedlichen aktiven Materialien gesprochen. Ein Masseanteil bezeichnet dabei den Beitrag des jeweiligen Materials zu der jeweiligen Gesamtmasse, das Masseverhältnis beschreibt den jeweiligen Masseanteil im Vergleich zu einem anderen Masseanteil. Beispielsweise kann das Masseverhältnis X:Y betrachtet werden, worin X für die Masse des die Reaktion von Wasserstoff, Kohlenstoffmonoxid und/oder Kohlenstoffdioxid zu Methanol katalysierenden Materials bzw. der Methanolsyntheskatalysatoren und Y für die Masse des katalytisch aktiven Materials für die Reaktion von Methanol zu Dimethylether und Wasser oder Dehydratisierungskatalysator steht.

Insbesondere weist das erste Katalysatorbett gegenüber dem zweiten Katalysatorbett einen höheren Anteil an katalytisch aktivem Material für die Reaktion von Wasserstoff, Kohlenstoffmonoxid und/oder Kohlenstoffdioxid zu Methanol auf. Dies kann durch den Einsatz eines reinen Methanolsynthesekatalysatorbettes im ersten Katalysatorbett erreicht werden, oder durch den Einsatz einer Mischung, in der der Anteil an katalytisch aktivem Material für die Reaktion von Wasserstoff, Kohlenstoffmonoxid und/oder Kohlenstoffdioxid zu Methanol höher ist, als jener der Reaktion von Methanol zu Dimethylether und Wasser. Letzteres ist bevorzugt, da somit der Gleichgewichtsumsatz höher liegt. Der erfindungsgemäß höhere Wärmeübergang in der ersten Reaktionszone ermöglicht nun, wie bereits beschrieben, das Einbringen eines höheren Anteils an katalytisch aktivem Material für die Reaktion von Wasserstoff, Kohlenstoffmonoxid und/oder Kohlenstoffdioxid zu Methanol und fördert somit zusätzlich eine höhere Ausnutzung der ersten Reaktionszone. Sozusagen ist in der ersten Reaktionszone nur ein Mindestanteil an katalytisch aktivem Material für die Reaktion von Methanol zu Dimethylether und Wasser notwendig um den höheren möglichen Gleichgewichtsumsatz auszunutzen. Das Massenverhältnis der aktiven Materialien liegt zwischen 1:1 bis 5:1 bevorzugt zwischen 3:1 und 5:1.

Im Gegenzug wird in der zweiten Reaktionszone die weniger stark exotherme Dehydratisierung und die durch den Mangel der Edukte kaum noch vorhandene Exothermie der Methanolsynthese und die daraus resultierende kinetische Verlangsamung der Gesamtreaktion berücksichtigt. Durch Gestaltung der zweiten Reaktionszone werden der Wärmeübergang und/oder die Linargeschwindigkeit verringert. Beide Umstände tragen dazu bei, dass die Ausnutzung der zweiten Reaktionszone erhöht wird. Zusätzlich kann die weniger stark exotherme Reaktion von Methanol zu Dimethylether und Wasser durch Erhöhen von deren Anteil an katalytisch aktivem Material zusätzlich unterstützt werden. Es sind lediglich Mindestmengen an katalytischem Material für die Reaktion von Wasserstoff mit Kohlenstoffmonoxid und/oder Kohlenstoffdioxid zu Methanol vorgesehen, um die Methanolsynthese als Eduktlieferant für die Dimethylethersynthese weiter am Laufen zu halten und somit möglichst nahe an den Gleichgewichtsumsatz zu gelangen. Das Massenverhältnis der aktiven Materialien liegt zwischen 0,6:1 bis 3:1 bevorzugt zwischen 1:1 und 3:1.

Insbesondere können beide Katalysatorbetten einen Gradienten in der Zusammensetzung aufweisen, so dass in einem eingangsseitig angeordneten Abschnitt des ersten Rohrteilbündels ein geringerer Anteil an katalytisch aktivem Material, das Wasserstoff, Kohlenstoffmonoxid und/oder Kohlenstoffdioxid zu Methanol umsetzt, vorliegt, als in einem ausgangsseitig angeordneten Abschnitt des jeweiligen Rohrteilbündels. Das hat den Vorteil, dass am Eintritt der ersten Reaktionszone eine zusätzliche Exothermiekontrolle existiert, da hier ohnehin hohe Eduktmengen auf hohe Katalysatormengen treffen. Für die zweite Reaktionszone ist dies von weiterem Vorteil, da hier, wie im späteren Verlauf erklärt, eine Einbindung des nicht konvertierten Methanolstromes vorgesehen ist. Somit sind am Eintritt der zweiten Reaktionszone höhere Mengen an katalytischem Material zur Umsetzung von Methanol zu Dimethylether und Wasser nötig. Weiter stromab in dieser zweiten Reaktionszone kann der Anteil an katalytischem Material für die Umsetzung von Synthesegas zu Methanol und Wasser wieder angehoben werden, um der Eduktverarmung, wie auch im Falle der ersten Reaktionszone, entgegenzukommen bzw. der Zwischenproduktverarmung (Methanol) und somit der Eduktverarmung der zweiten Reaktion entgegenzuwirken.

Mit anderen Worten überwiegt in der ersten Reaktionszone der Anteil an katalytisch aktivem Material im Katalysatorbett, das Wasserstoff, Kohlenstoffmonoxid und/oder Kohlenstoffdioxid zu Methanol umsetzt gegenüber der zweiten Reaktionszone und innerhalb einer Reaktionszone kann dieser Anteil einen Gradienten besitzen, so dass der Massenanteil des Methanolsynthesekatalysators eintrittsseitig niedriger ist und austrittsseitig höher.

Der Gradient kann technisch in Teilzonen mit unterschiedlichen Mischungen gestaltet sein. Jede Reaktionszone kann beispielsweise in drei Teilzonen eingeteilt werden, die mit jeweils anderer Mischung bzw. Schüttung des aktiven Materials beaufschlagt werden. Dabei kann sich die Katalysatorzusammensetzung in den ersten 15-30% des Rohres gegenüber dem hinteren Bett zwischen 50 und 100% der Rohrlänge unterscheiden. In der Teilzone dazwischen kann eine weitere Teilzone mit unterschiedlicher Mischung vorliegen bzw. kann diese der vorderen oder hinteren gleichartig sein. Dies hängt von den jeweiligen Wärmetransporteigenschaften des Rohres ab. Jedoch haben alle Schüttungen gemeinsam, dass der Anteil an katalytisch aktivem Material für die Reaktion von Wasserstoff, Kohlenstoffmonoxid und/oder Kohlenstoffdioxid zu Methanol eintrittsseitig nach austrittseitig aufsteigend nach oben genannten Grundsätzen angeordnet ist.

Gemeinsamer Vorteil aller dieser Ausgestaltungen ist die homogenere Verteilung der Reaktion über die gesamte Reaktorlänge und gezielter örtlicher Einsatz von Katalysatoren und damit eine erhöhte Steuerbarkeit, Ausnutzung des Bettes und verminderte temperaturbedingte Katalysatoralterung.

In einer besonderen alternativen Ausgestaltung können anstelle von Mischbetten in der ersten Reaktionszone die unterschiedlichen aktiven Materialien in Schichten angeordnet werden, so dass das gesamte in der ersten Reaktionszone vorgesehene katalytisch aktive Material für die Reaktion von Wasserstoff, Kohlenstoffmonoxid und/oder Kohlenstoffdioxid zu Methanol eintrittsseitig der ersten Reaktionszone vorliegt und das ganze in der ersten Reaktionszone vorgesehene katalytische Material für die Reaktion von Methanol zu Dimethylether und Wasser austrittsseitig der ersten Reaktionszone. Dies birgt den Vorteil, dass entstehendes Wasser der Dehydratisierung vor Eintritt in die zweite Reaktionszone abgetrennt werden kann. Dieses Wasser kann dort dann nicht mehr mit Kohlenstoffmonoxid zu Kohlenstoffdioxid und Wasserstoff umgesetzt werden. Somit steigert diese Anordnung zusätzlich die Kohlenstoffausbeute des Prozesses und ist besonders für wasserstoffreiche Synthesegase eine relevante Anordnung. Dies bedingt eine zusätzliche Trennung der ersten Reaktionszone austrittseitig und zweiten Reaktionszone eintrittsseitig, und eine Verbindung zur Produktaufbereitung. Die erläuterten Unterschiede der gesamten Anteile zwischen der ersten und der zweiten Reaktionszone bleiben dabei jedoch weiterhin bestehen.

In vorteilhaften Ausgestaltungen des Reaktors ist die erste Reaktionszone dazu eingerichtet, in einer ersten Richtung durchströmt zu werden und die zweite Reaktionszone dazu eingerichtet, in einer zweiten Richtung, die vorzugsweise antiparallel zu der ersten Richtung ist, durchströmt zu werden. Durch die Richtungsumkehr kann einerseits ein kompakteres Reaktordesign realisiert werden, andererseits kann eine bessere Homogenisierung eines Zwischenproduktstroms, der die erste Reaktionszone verlässt, vor Eintritt in die zweite Reaktionszone erreicht werden, insbesondere da sich Ströme aus unterschiedlichen Rohren des ersten Rohrbündels unter Verwirbelung, die durch die Richtungsumkehr begünstigt wird, miteinander mischen können.

Der Mantelraum austrittsseitig der ersten Reaktionszone und der Mantelraum eintrittsseitig der zweiten Reaktion können auch derart strömungstechnisch verbunden sein, so dass auch parallellaufende Strömungsrichtungen der unterschiedlichen Reaktionszonen möglich sind. Dies wäre ein zusätzlicher apparativer Aufwand über entsprechende Rohrleitungen, hat aber den Vorteil, dass gegebenenfalls eine Fluidisierung des Katalysators und der mit ihr verbundene Austrag aus dem Reaktor und/oder eine Entmischung der Katalysatormischung unterbunden werden kann.

Jede der Reaktionszonen des Rohrbündelreaktors ist vorteilhafterweise dazu eingerichtet, mantelseitig von einem Kühlmedium umströmt zu werden, um die Reaktionswärme effizient abzuführen. Dabei kann ein gemeinsamer Kühlmantel für die erste und die zweite Reaktionszone vorgesehen sein, oder voneinander separierte Kühlmäntel. Ersteres bietet den Vorteil eines einfacheren Reaktordesigns (und damit geringere Investitionskosten), letzteres den Vorteil einer präziseren Reaktionssteuerung (und damit ggf. erhöhte Produktausbeute und/oder -qualität; gezielter Einfluss auf unterschiedliche Katalysatoralterungen in den Betten, da Methanolsynthesekatalysatoren unter reduzierenden Bedingungen (erstes Katalysatorbett) langsamer altern als unter oxidierenden Bedingungen (zweites Katalysatorbett). Durch die erfindungsgemäße Gestaltung der Reaktorzonen kann insbesondere auf unterschiedliche Kühlsysteme für die Reaktorzonen verzichtet werden, um Einfluss auf die jeweils dominierend vorliegende Reaktion zu nehmen und somit zu Teilen beide Vorteile genutzt werden.

Im Rahmen der Erfindung zum Einsatz kommende Kühlmittel sind typischerweise Siedewasser, Thermoöle oder Salzschmelzen. Ihre Wahl ist vom Temperatureinsatz und Art der Verwendung der abgeführten Wärme abhängig. Im Falle einer Direktsynthese zu Dimethylether kann in Prinzip ein gemeinsames Kühlmedium für beide Reaktionszonen eingesetzt werden. Es können Unterschiede zwischen der Anströmrichtung und Anströmgeschwindigkeit in der ersten Reaktionszone gegenüber zweiten Reaktionszone realisiert werden, um Einfluss auf den äußeren Wärmeübergang zu nehmen. Dies kann vorteilhaft sein, um die Rohrbündelgeometrien möglichst symetrisch zu gestalten. In der Regel wird jedoch auf einen hohen äußeren Wärmeübergang geachtet. Für andere Methanolfolgeprodukte können auch zwei unterschiedliche Kühlmedien zum Einsatz kommen, um eventuell unterschiedliche Temperaturanforderungen abzudecken. Die Direktsynthese von Olefinen aus Synthesegas benötigt z.B. Temperaturen zwischen 300-400°C, während die Direktsynthese von Dimethylether im Bereich von 200-300°C durchgeführt wird. Eine Kombination beider Prozesse kann zu zwei unterschiedlichen Kühlmedien und Kreisläufen führen.

Ein erfindungsgemäßes Verfahren profitiert in entsprechenden Ausgestaltungen in analoger Weise von den in Bezug auf den Reaktor erläuterten Vorteilen, so dass diese lediglich aus Gründen der besseren Lesbarkeit hier nicht wiederholt dargestellt werden sollen, auf die jedoch ausdrücklich verwiesen wird.

Im Nachfolgenden sollen an einem Beispiel die Vorteile einer Ausführung der Erfindung verdeutlicht werden:
Ein zwei Meter langes Rohr mit 24,8mm Innendurchmesser wurde mit einer Mischung aus einem Methanolsynthesekatalysator und einem Dehydratisierungskatalystor im Verhältnis 1:1,11 gefüllt und einem Synthesegas ausgesetzt. Reaktionsbedingungen und erzielte Konversionen sind aus der Tabelle zu entnehmen.

Das Temperaturprofil aller Versuche verdeutlicht, dass in den ersten 40% des Bettes eine höhere Energieabfuhr nötig ist als im restlichen Bett. Vergleicht man Experiment 1 und Experiment 2, wird deutlich, dass eine verbesserte Energieabfuhr alleine durch die Erhöhung der Raumgeschwindigkeit von 2000h⁻¹ auf 3000h⁻¹ zu Verlusten in der Gesamtkonversion zu DME führt. Alle Versuche zeigen, dass die Konversion von Kohlenmonoxid nicht am möglichen Gleichgewichtsumsatz (>90%) und somit im wirtschaftlich interessantesten Bereich durchgeführt werden konnten. Um höhere Umsätze zu erreichen, ist eine Erhöhung des Methanolsynthesekatalystorsanteils nötig oder eine Reduzierung der Raumgeschwindigkeit. Beides ist aufgrund der Temperatureinschränkungen des verwendeten Katalysatorsystems nicht möglich. Zusätzliches Einbringen von Methanolsynthesekatalysatoren im hinteren Teil des Rohres kann die Konversion von Kohlenstoffmonoxid erhöhen. Diese Erkenntnis ist die Basis für ein Bett mit Gradienten, in dem der Methanolsynthesekatalysatoranteil von vorne nach hinten ansteigend angeordnet ist. Alternativ wäre ein höherer Anteil an Methanolsynthesekatalysator durch Verbesserung des Wärmeüberganges möglich, dies würde in diesem Fall bei gleicher Rohrlänge und Katalysatorvolumen zu dünneren und mehreren Rohren führen.

Die erfindungsgemäße Ausgestaltung des Reaktors überwindet die oben beschriebenen Probleme.

Im Experiment 3 wurde ein Methanolrecycle realisiert. Es zeigte sich, dass das eingetragene Methanol dehydratisiert wird und die Konversion von Kohlenstoffmonoxid gegenüber der Referenz (Experiment 2) aufrechterhalten bleibt. Somit kann theoretisch Dehydratisierungskatralysator durch Methanolsynthesekatalysator ersetzt werden, um höhere Ausbeuten zu erreichen. Dies kann durch höheren Methanolsynthesekatalysatoranteil in der ersten Reaktionszone der Fall sein und/oder durch Einbringen eines Gradienten, wie bereits beschrieben.

In Experiment 4 wurde eine der einfachsten Möglichkeiten der Zwischenabtrennung zwischen den beiden Reaktionszonen abgebildet. Der anfallende Effluent wurde bei 10°C auskondensiert und das nicht umgesetzte Synthesegas zum Teil wieder vor den Reaktor gefahren.

| Experiment | [-] | 1 | 2 | 3 | 4 |
|---|---|---|---|---|---|
| TOS | [h] | 171 | 193 | 268 | 505 |
| pressure | Bara | 60 | 60 | 60 | 60 |
| GHSV | GHSV | 2002 | 3002 | 2980 | 2174 |

| Composition to reactor inlet | | | | | |
|---|---|---|---|---|---|
| x H₂ | mol/mol | 0,508 | 0,510 | 0,514 | 0,534 |
| x CO | mol/mol | 0,253 | 0,252 | 0,255 | 0,156 |
| x CO₂ | mol/mol | 0,040 | 0,040 | 0,040 | 0,094 |
| x N₂ | mol/mol | 0,200 | 0,198 | 0,170 | 0,195 |
| x O₂ | mol/mol | 0,000 | 0,000 | 0,000 | 0,000 |
| x DME | mol/mol | 0,000 | 0,000 | 0,000 | 0,020 |
| x MeOH | mol/mol | 0,000 | 0,000 | 0,022 | 0,000 |

| CO Conversion and Selectivities based on reactor in/out | | | | | |
|---|---|---|---|---|---|
| x CO | mol/mol | 82% | 71% | 70% | 74% |
| S CO₂ | mol C/mol CO | 27% | 25% | 27% | 16% |
| S DME | mol C/mol CO | 60% | 56% | 65% | 57% |
| S MeOH | mol C/mol CO | 12% | 18% | 7% | 27% |
| S Others | mol C/mol CO | 0,7% | 0,7% | 0,7% | 0,5% |

| Temperature profiles in °C | | | | | |
|---|---|---|---|---|---|
| TI XXX | length [mm] | °C | | | |
| TIR 302 | 195 | 251 | 251 | 251 | 261 |
| TIR 303 | 345 | 282 | 280 | 275 | 280 |
| TIR 304 | 495 | 282 | 282 | 279 | 280 |
| TIR 305 | 645 | 275 | 277 | 275 | 275 |
| TIR 306 | 795 | 275 | 278 | 277 | 275 |
| TIR 307 | 945 | 268 | 271 | 271 | 272 |
| TIR 308 | 1145 | 264 | 268 | 267 | 269 |
| TIR 309 | 1345 | 263 | 268 | 268 | 268 |
| TIR 310 | 1545 | 260 | 264 | 264 | 266 |
| TIR 311 | 1745 | 257 | 261 | 261 | 265 |
| TIR 335 | Oil inlet | 250 | 250 | 250 | 260 |
| TIR 338 | Oil outlet | 251 | 251 | 251 | 261 |

Die Erfindung wird unter Bezugnahme auf die Zeichnungen näher erläutert, die Ausführungsformen der Erfindung zeigen.

### Kurze Beschreibung der Zeichnungen

Figur 1 zeigt einen Reaktor zur Erzeugung von Dimethylether gemäß einer ersten Ausführungsform der Erfindung in schematischer Darstellung.
Figur 2 zeigt einen Reaktor zur Erzeugung von Dimethylether gemäß einer zweiten Ausführungsform der Erfindung in schematischer Darstellung.
Figur 3 zeigt einen Reaktor zur Erzeugung von Dimethylether gemäß einer dritten Ausführungsform der Erfindung in schematischer Darstellung.
Figur 4 zeigt eine Anordnung mit einem Reaktor zur Erzeugung von Dimethylether gemäß einer Ausführungsform der Erfindung in schematischer Darstellung.
Figur 5 zeigt eine Anordnung von Reaktoren zur Erzeugung von Dimethylether mit Merkmalen der Erfindung in schematischer Darstellung.

In den Figuren sind einander entsprechende Elemente mit identischen Bezugszeichen angegeben und werden der Übersichtlichkeit halber nicht wiederholt erläutert.

### Ausführliche Beschreibung der Zeichnungen

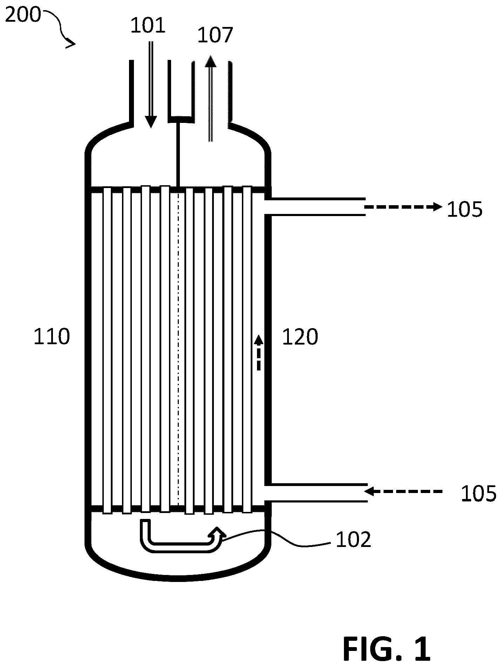

In Figur 1 ist ein Reaktor zur Erzeugung von Dimethylether gemäß einer ersten Ausführungsform der Erfindung schematisch gezeigt und insgesamt mit 200 bezeichnet, bei dem die erste 110 und zweite 120 Reaktionszone (anti)parallel zueinander und nebeneinander angeordnet sind. Dementsprechend sind das erste und das zweite Katalysatorbett räumlich voneinander getrennt, zudem kann ein Rohrdurchmesser in der ersten Reaktionszone 110 anders gewählt werden als ein Rohrdurchmesser in der zweiten Reaktionszone 120 und/oder das Volumen der Reaktionszone 110 anders gewählt sein als das Volumen der zweiten Reaktionszone (120)Dies hat, wie eingangs bereits erwähnt den Vorteil, dass insbesondere in der ersten Reaktionszone 110 ein erhöhter Kühlbedarf besteht, da die dort ablaufende Reaktion stark exotherm ist. Daher werden idealerweise in der ersten Reaktionszone Rohre 140 eingesetzt, die einen geringeren Durchmesser aufweisen, als Rohre 140 in der zweiten Reaktionszone 120. Beispielsweise können in der ersten Reaktionszone 110 Rohre mit einem Rohrinnendurchmesser im Bereich von 2 bis 6 cm, beispielsweise herkömmliche zollige Rohre 140 (mit einem Durchmesser von etwa 2,4 cm) eingesetzt werden, während in der zweiten Reaktionszone beispielsweise Rohre 140 mit einem Rohrdurchmesser im Bereich von 2 bis 8 cm, z.B. zweizollige Rohre (mit einem Durchmesser von ca. 4,8 cm) vorgesehen werden. Insgesamt ergibt sich somit bei gleichen Volumen der beiden Zonen eine höhere Wärmeaustauschoberfläche in der ersten Reaktionszone oder bei einem kleineren Volumen der ersten Reaktionszone eine höhere Lineargeschwindigkeit des Fluids in der ersten Reaktionszone, was zu einer höheren Kühlleistung führt.

Alternativ werden für beide Reaktionszonen 110 und 120 gleiche Rohrdurchmesser verwendet. So sind entsprechend weniger Rohre in der ersten Reaktionszone 110 als in der zweiten Reaktionszone 120 anzuordnen. Dadurch werden in der ersten Reaktionszone 110 höhere Raumgeschwindigkeit als in der zweiten Reaktionszone 120 erzielt und somit eine höhere Wärmeabfuhr.

Dadurch kann in der ersten Reaktionszone 110 mehr katalytisch aktives Material für die Methanolsynthese untergebracht werden und die hauptsächlich ablaufende Reaktion zu Methanol kontrolliert werden, was sich positiv auf die Katalysatorlebensdauer auswirkt, da die entstehende Wärme effizient aufgenommen werden kann und somit lokale Temperaturspitzen vermieden bzw. verringert werden und die höhere eingebrachte Menge an aktiven Material für die Methanolsynthese resistenter gegen Katalysatorgifte und Deaktivierungen ist.

Durch die parallele und insbesondere gegenströmend nebeneinanderliegende Anordnung der ersten 110 zu der zweiten 120 Reaktionszone ergibt sich, dass ein Zwischenproduktstrom 102, der die erste Reaktionszone verlässt, eine Richtungsänderung erfährt, bevor er in die zweite Reaktionszone 120 eingespeist wird. Durch diese Richtungsänderung ergeben sich Verwirbelungen, die zu einer Homogenisierung des Zwischenproduktstroms 102 beitragen, was sich vorteilhaft auf die Zusammensetzung des Rohproduktstroms 107 auswirkt. Zudem kann (nicht dargestellt) in den Strom 102 zusätzlich ein Eduktstrom eingebracht werden, vorteilhafterweise zumindest teilweise bestehend aus nicht konvertiertem Methanol aus dem Rohproduktstrom 107.

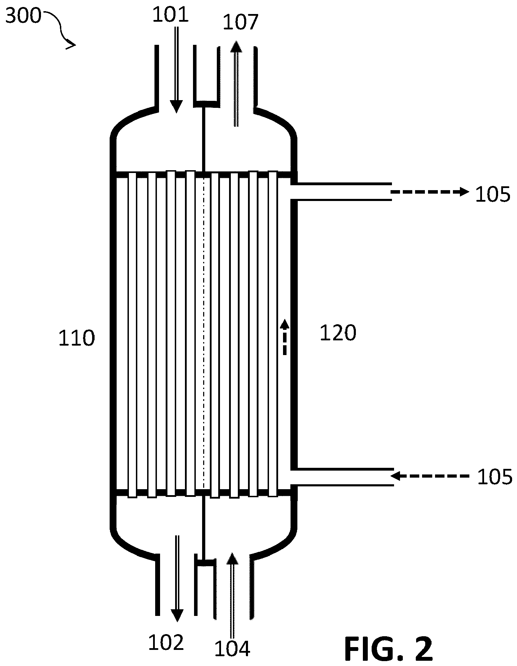

Eine zweite Ausführungsform eines erfindungsgemäßen Reaktors ist in Figur 2 schematisch dargestellt und insgesamt mit 300 bezeichnet. Der hier gezeigte Reaktor 300 unterscheidet sich von dem in Bezug auf Figur 1 beschriebenen Reaktor 200 insbesondere dadurch, dass hier eine Entnahme des Zwischenproduktstroms 102 vorgesehen ist. Dies ist insbesondere vorteilhaft, da so eine Zwischenaufarbeitung des Zwischenproduktstroms 102 ermöglicht wird, was sich positiv auf die Steuerung des Betriebs des Reaktors bzw. auf die erzielbare Produktausbeute und -qualität auswirken kann. Gegebenenfalls können bestimmte Komponenten des Zwischenproduktstroms 102 abgetrennt und aus der Anlage ausgeschleust oder an anderer Stelle dem Reaktor 300 wieder zugeführt werden. Zumindest ein Teil des derart entnommenen Zwischenproduktstroms 102 wird als Zwischeneduktstrom 104 in die zweite Reaktionszone 120 eingespeist.

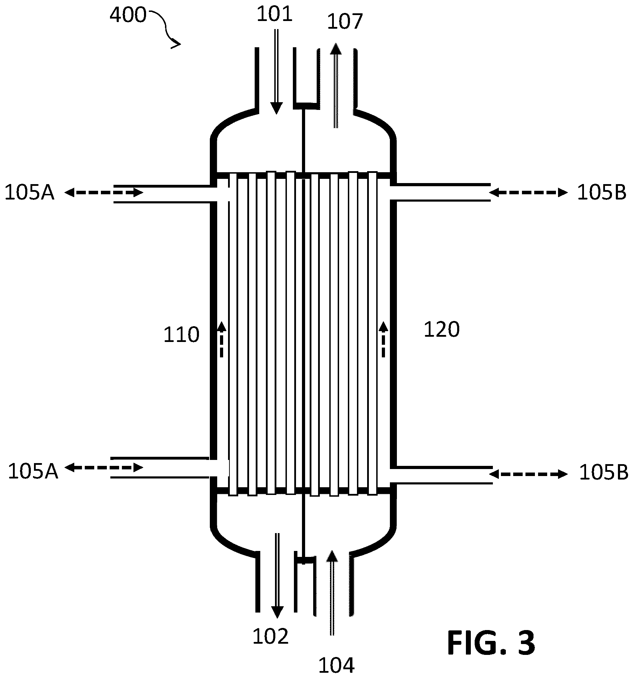

In Figur 3 ist eine dritte Ausgestaltung eines erfindungsgemäßen Reaktors dargestellt und insgesamt mit 400 bezeichnet. Von dem Reaktor 300, der in Bezug auf Figur 2 erläutert wurde, unterscheidet sich der in Figur 3 dargestellte Reaktor 400 insbesondere durch eine separate Kühlung der ersten 110 und zweiten 120 Reaktionszonen. Dadurch kann einerseits, wie in der Figur dargestellt in beiden Reaktionszonen 110, 120 eine Gegenstromkühlung realisiert werden, andererseits können die beiden Zonen auch auf unterschiedliche Temperaturen temperiert werden, so dass für beide Reaktionen jeweils möglichst optimale Reaktionsbedingungen eingestellt werden können. Dazu werden zwei Kühlmittel 105A und 105B bereitgestellt, die optional in voneinander separaten Kühlkreisläufen geführt werden. Es kann jedoch auch vorgesehen sein, dass die beiden Kühlmittel 105A, 105B in einem gemeinsamen Kühlkreislauf geführt werden, wobei beide Kühlmittel 105A, 105B beispielsweise in ihrer jeweils in den Mantelbereich 130 eingespeisten Menge separat gesteuert werden können.

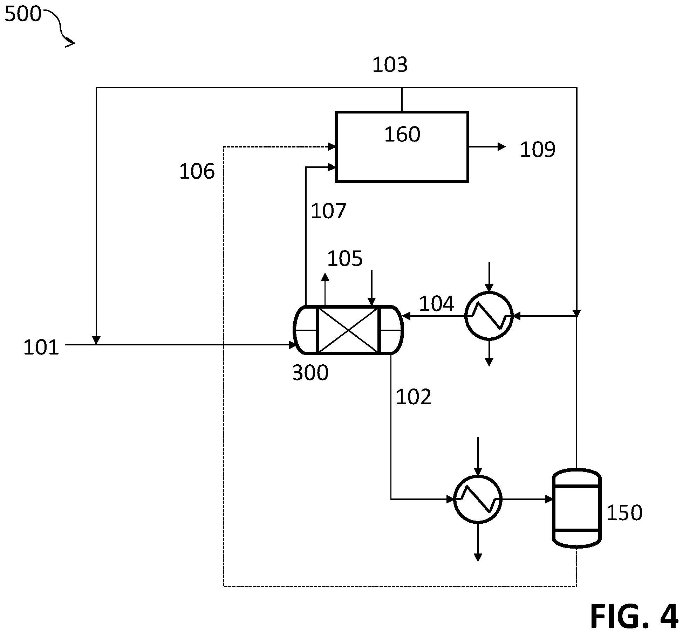

In Figur 4 ist eine Anordnung mit einer Ausführungsform eines erfindungsgemäßen Reaktors dargestellt und insgesamt mit 500 bezeichnet. Der hier dargestellte Reaktor 300 entspricht dem Reaktor, der in Bezug auf Figur 4 beschrieben wurde. Es kann jedoch auch ein anderer Reaktor an seiner statt verwendet werden, beispielsweise ein Reaktor 400 der dritten Ausführungsform.

Die Anordnung 500 umfasst neben dem Reaktor 300 einen Zwischenseparator 150, der beispielsweise dazu eingerichtet ist, den Zwischenproduktstrom 102 aus der ersten Reaktionszone 110 an bestimmten Komponenten abzureichern. Beispielsweise kann der Zwischenseparator 150 einen Gasabscheider und/oder eine Destillationsvorrichtung und/oder einen Absorber und/oder einen Adsorber umfassen, wobei auch andere Trennvorrichtungen zum Einsatz kommen können. Insbesondere kann der Zwischenseparator dazu eingerichtet sein, bei den in dem Zwischenseparator eingestellten Bedingungen kondensierbare Komponenten 106 einer Produktaufbereitung in dem Produktseparator 160 zuzuführen und/oder leichte bzw. niedrigsiedende Komponenten 104 in die zweite Reaktionszone 120 weiterzuführen. In der Produktaufbereitung 160 werden Produkte 109 Edukte und Zwischenprodukte 103 getrennt und können gezielt den jeweiligen Reaktionszonen 110, 120 zugeführt werden.

Der Produktseparator 160 kann insbesondere dazu eingerichtet sein, aus dem Rohproduktstrom 107 und den höher siedenden Komponenten 106, die aus dem Zwischenproduktstrom abgetrennt wurden, einen an Dimethylether reichen Produktstrom 109 zu bilden. Ferner kann der Produktseparator 160 dazu eingerichtet sein, einen oder mehrere Recyclingströme 103 bereitzustellen, die insbesondere Synthesegaskomponenten und/oder Methanol enthalten und dementsprechend an geeigneten Stellen in den Reaktor 300 zurückgeführt werden. Insbesondere werden dabei Synthesegaskomponenten zusammen mit dem Einsatzstrom 101 in die erste Reaktionszone 110 des Reaktors 300 zurückgeführt, während Methanol bevorzugt in die zweite Reaktionszone 120 des Reaktors 300 zurückgespeist wird. Es versteht sich, dass beispielsweise Wärmetauscher, Ventile, Druckminderer, Verdichter und weitere Vorrichtungen zur Beeinflussung der Reaktionsbedingungen an geeigneter Stelle der Anordnung bereitgestellt sein können, ohne hier explizit erläutert zu werden. Das Trennprinzip des Produktseparators 160 kann insbesondere auf unterschiedlichen Siedepunkten, Ad- und/oder Absorptionsverhalten, Membrandurchgängigkeit, Polarität oder anderen physikalisch-chemischen Eigenschaften der Einzelverbindungen basieren. Auch eine Kombination solcher Trennprinzipien kann gegebenenfalls vorteilhaft sein.

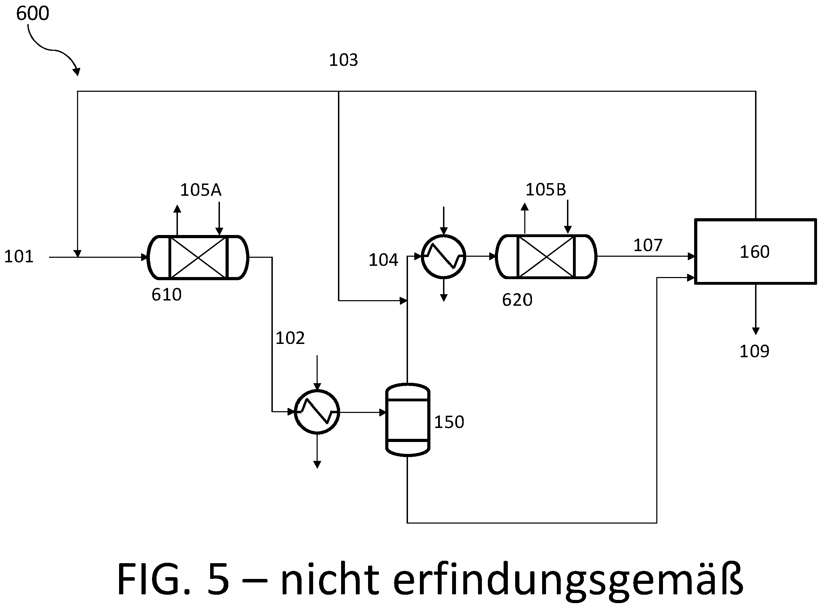

Zum Vergleich ist in Figur 5 eine alternative Anordnung dargestellt und insgesamt mit 600 bezeichnet. Die Anordnung 600 unterscheidet sich insbesondere dadurch von den bisher erläuterten Anordnungen, dass mehrere separate Reaktoren 610, 620 vorgesehen sind, in denen jeweils eine einzige Reaktion katalysiert wird, wobei Kühlmittel 105A, 105B jeweils mantelseitig in die Reaktoren 610 bzw. 620 eingespeist werden. Es sei ausdrücklich betont, dass auch in einer solchen Anordnung 600 Merkmale der Erfindung realisiert sein können. So kann der Reaktor 610 die erste Reaktionszone (110) aufnehmen und der Reaktor 620 die zweite Reaktionszone (120) aufnehmen und beide Reaktoren einen gemeinsamen Kühlkreislauf besitzen. Im Vergleich dazu bieten die zuvor diskutierten Anordnungen 200, 300, 400, 500 eine erhebliche Reduktion der Investitionskosten durch kompakter gestaltete Reaktoren und kürzere Rohrleitungen außerhalb der Reaktoren, wodurch sich auch die Betriebskosten senken lassen, da beispielsweise geringere Kühlmittelmengen bereitgestellt und bearbeitet werden müssen.

Vorteilhafte Ausgestaltungen erfindungsgemäßer Verfahren verwenden insbesondere Reaktoren, wie sie hier beschrieben wurden und profitieren daher dementsprechend von deren Vorteilen. Die in Bezug auf Ausgestaltungen erfindungsgemäßer Reaktoren erläuterten Merkmale gelten daher sinngemäß entsprechend auch für jeweils entsprechende Verfahren.

Es sei ausdrücklich betont, dass die beschriebenen Merkmale nicht allein in der jeweils beschriebenen Kombination, sondern auch in anderen Kombinationen sowie in Alleinstellung ihre jeweiligen Vorteile entfalten können, so dass die hier beschriebenen Kombinationen lediglich als Beispiele zum besseren Verständnis der Erfindung zu verstehen sind.

## Patentansprüche

**1.** Reaktor (200, 300, 400) zur katalytischen Herstellung von Methanolfolgeprodukten, insbesondere Dimethylether, aus Synthesegas, der als Rohrbündelreaktor mit einem aus Rohren (140) gebildeten Rohrbündel und zumindest einem, die Rohre umgebenden Mantel, der einen eingangsseitigen und einen ausgangsseitigen Rohrboden aufweist, wobei die Rohrböden einen Gasraum, der die Rohre (140) umfasst, von einem Kühlmedienraum trennen, ausgestaltet ist und zumindest eine erste Reaktionszone (110) und eine zweite (120) Reaktionszone aufweist, wobei die erste Reaktionszone (110) mit einem ersten Katalysatorbett beaufschlagt ist und die zweite Reaktionszone (120) mit einem zweiten Katalysatorbett beaufschlagt ist, im Betrieb die zweite Reaktionszone (120) stromab der ersten Reaktionszone (110) liegt, das erste und das zweite Katalysatorbett dazu eingerichtet sind, eine Direktsynthese umfassend eine Reaktion von Wasserstoff mit Kohlenstoffmonoxid und/oder Kohlenstoffdioxid zu Methanol und eine Reaktion von Methanol zu einem oder mehreren Methanolfolgeprodukten und Wasser zu katalysieren,
**dadurch gekennzeichnet, dass**
das erste Katalysatorbett dazu eingerichtet ist, überwiegend die Reaktion von Wasserstoff mit Kohlenstoffmonoxid und/oder Kohlenstoffdioxid zu Methanol zu katalysieren und das zweite Katalysatorbett dazu eingerichtet ist, überwiegend die Reaktion von Methanol zu einem oder mehreren Methanolfolgeprodukten und Wasser zu katalysieren, wobei der Massenanteil des katalytisch aktiven Materials für die Reaktion von Wasserstoff mit Kohlenstoffmonoxid und/oder Kohlenstoffdioxid zu Methanol an dem ersten Katalysatorbett in der ersten Reaktionszone den Massenanteil des katalytisch aktiven Materials für die Reaktion von Wasserstoff mit Kohlenstoffmonoxid und/oder Kohlenstoffdioxid zu Methanol an dem zweiten Katalysatorbett überschreitet,
die erste Reaktionszone (110) für einen Betrieb mit einem ersten volumenbezogenen Wärmeübergang von der Reaktionszone auf ein die erste Reaktionszone (110) umgebendes Kühlmedium (105, 105A) und die zweite Reaktionszone (120) für einen Betrieb mit einem zweiten volumenbezogenen Wärmeübergang von der Reaktionszone auf ein die zweite Reaktionszone (120) umgebendes Kühlmedium (105, 105B) ausgebildet ist.

**2.** Reaktor (200, 300, 400) nach Anspruch 1, wobei der erste Wärmeübergang den zweiten Wärmeübergang übersteigt.

**3.** Reaktor (200, 300, 400) nach Anspruch 1 oder 2, wobei ein Unterschied zwischen dem ersten und dem zweiten Wärmeübergang im Betrieb durch Unterschiede der ersten Reaktionszone (110) und der zweiten Reaktionszone (120) bezüglich Volumen und/oder Innendurchmesser der Rohre (140), und/oder Anströmgeschwindigkeit des Kühlmediums (105, 105A, 105B) und/oder Anströmrichtung des Kühlmediums (105, 105A, 105B) eingestellt wird.

**4.** Reaktor (200, 300, 400) nach einem der vorstehenden Ansprüche, wobei die erste Reaktionszone (110) in den Rohren (140) eines ersten Rohrteilbündels ausgebildet ist, die zweite Reaktionszone (120) in den Rohren (140) eines zweiten Rohrteilbündels ausgebildet ist, und das erste Rohrteilbündel (110) und das zweite Rohrteilbündel (120) in mindestens einem Mantelraum angeordnet sind.

**5.** Reaktor (200, 300, 400) nach Anspruch 4, wobei Rohre des ersten Rohrteilbündels zumindest in einem Abschnitt parallel neben Rohren des zweiten Rohrteilbündels verlaufen, die erste Reaktionszone (110) in den Rohren des ersten Rohrteilbündels ausgebildet ist, die zweite Reaktionszone (120) in den Rohren des zweiten Rohrteilbündels ausgebildet ist, und die Rohre des ersten Rohrteilbündels und die Rohre des zweiten Rohrteilbündels in einem gemeinsamen Mantelraum angeordnet sind.

**6.** Reaktor (200, 300, 400) nach einem der vorstehenden Ansprüche, wobei das Volumen des Katalysatorbettes der ersten Reaktionszone (110) das 0,3 bis 0,75 fache des Volumens des Katalysatorbettes der zweiten Reaktionszone (120) beträgt.

**7.** Reaktor (200, 300, 400) nach einem der vorhergehenden Ansprüche, wobei der Innendurchmesser der Rohre (140) aus einem Bereich von 20 mm bis 80 mm und bevorzugt der Innendurchmesser der Rohre (140) der ersten Reaktionszone (110) aus einem Bereich von 20 mm bis 60 mm und der Innendurchmesser der Rohre (140) der zweiten Reaktionszone (120) aus einem Bereich von 20 mm bis 80 mm ausgewählt ist, wobei der Innendurchmesser der Rohre (140) der ersten Reaktionszone (110) den Innendurchmesser der Rohre (140) der zweiten Reaktionszone (120) nicht überschreitet.

**8.** Reaktor (200, 300, 400) nach einem der vorhergehenden Ansprüche, wobei der Gasraum des Mantels gasausgangsseitig der ersten Reaktionszone (110) und der Gasraum des Mantels gaseingangsseitig der zweiten Reaktorzone (120) miteinander strömungstechnisch verbunden ist und wobei der Gasraum stromauf der zweiten Reaktionszone (120) eine Zufuhröffnung für einen Zwischeneduktstrom (104) aufweist.

**9.** Reaktor (200, 300, 400) nach einem der vorhergehenden Ansprüche wobei der Gasraum des Mantels gasausgangsseitig der ersten Reaktionszone (110) vom Gasraum des Mantels gaseingangsseitig der zweiten Reaktionszone innerhalb des Mantels strömungstechnisch getrennt ist und gasausgangsseitig der ersten Reaktionszone (110) eine Entnahmestelle zur Entnahme eines Zwischenproduktstroms (102) aufweist.

**10.** Reaktor (200, 300, 400) nach einem der vorhergehenden Ansprüche, wobei zumindest eines der ersten und zweiten Katalysatorbetten eine Mischung aus mindestens einem Methanolsynthesekatalysator und mindestens einem Dehydratisierungskatalysator aufweisen.

**11.** Reaktor (200, 300, 400) nach Anspruch 10 wobei ein Masseverhältnis von Methanolsynthesekatalysator zu Dehydratisierungskatalysator des ersten Katalysatorbetts das Masseverhältnis von Methanolsynthesekatalysator zu Dehydratisierungskatalysator des zweiten Katalysatorbetts überschreitet, wobei insbesondere das Masseverhältnis in der ersten Reaktionszone zwischen 1:1 und 5:1 und das Masseverhältnis in der zweiten Reaktionszone zwischen 0,6:1 und 3:1 liegt.

**12.** Reaktor (200, 300, 400) nach Anspruch 10 oder 11, wobei das Masseverhältnis von Methanolsynthesekatalysator zu Dehydratisierungskatalysator im ersten Katalysatorbett und/oder im zweiten Katalysatorbett einen Gradienten aufweist, so dass in einem eingangsseitig angeordneten Abschnitt der jeweiligen Reaktionszone ein geringerer Anteil an Methanolsynthesekatalysator vorliegt, als in einem ausgangsseitig angeordneten Abschnitt der jeweiligen Reaktionszone.

**13.** Reaktor (200, 300, 400) nach Anspruch 10, 11 oder 12, wobei der verhältnismäßige Massenanteil aus einem Methanolsynthesekatalysator und einem Dehydratisierungskatalysator im Katalysatorbett der ersten Reaktionszone (110) als voneinander getrennte Katalysatorteilbetten vorliegt und im Katalysatorbett der zweiten Reaktionszone (120) als Mischung vorliegt, wobei ausgangsseitig der ersten Reaktionszone (110) das katalytisch aktive Material der Dehydratisierung und eingangsseitig der ersten Reaktionszone das katalytisch aktive Material der Methanolsynthese vorliegt.

**12.** Verfahren zur Herstellung Methanolfolgeprodukten und insbesondere Dimethylether aus Synthesegas unter Verwendung eines Reaktors (200, 300, 400) nach einem der Ansprüche 1 bis 13.

**15.** Verfahren nach Anspruch 14, wobei ein Druck innerhalb der ersten (110) und der zweiten (120) Reaktionszonen auf ein Druckniveau in einem Bereich von 1 bis 10 MPa, bevorzugt zwischen 3 und 10 MPa, eine Temperatur innerhalb der ersten (110) und/oder zweiten (120) Reaktionszone auf ein Temperaturniveau in einem Bereich von 200 bis 450 °C, bevorzugt zwischen 200 und 300 °C und eine GHSV bezogen auf das gesamte Katalysatorvolumen zwischen 2000 und 5000 h⁻¹ eingestellt wird.

**16.** Verfahren nach Anspruch 12 oder 13, wobei aus dem Reaktor (200, 300, 400) stromab der zweiten (120) Reaktionszone Methanol, Wasser und nicht umgesetztes Synthesegas aus einem dem Reaktor (200, 300, 400) entnommenen Produktstrom (107) abgetrennt werden, CO₂ aus dem nicht umgesetzten Synthesegas zumindest zum Teil abgetrennt wird, das abgetrennte Methanol und nicht umgesetzte Synthesegas in den Reaktor (200, 300, 400) zurückgeführt wird, wobei das Methanol stromab der ersten Reaktionszone (110) in den Reaktor (100, 200, 300, 400) zurückgeführt wird, das abgetrennte nicht umgesetzte Synthesegas stromauf der ersten Reaktionszone (110) in den Reaktor (100, 200, 300, 400) zurückgeführt wird und das abgetrennte Wasser und CO₂ aus dem Verfahren ausgeschleust werden.

**17.** Verfahren nach Anspruch 14, 15 oder 16, wobei stromab der ersten (110) Reaktionszone dem Reaktor (200, 300, 400) ein Zwischenproduktstrom (102) entnommen, dieser mindestens zu einem Teil an Wasser abgereichert wird und der an Wasser abgereicherte Zwischenproduktstrom (102) stromauf der zweiten Reaktionszone (120) als Zwischeneduktstrom (104) in den Reaktor (200, 300, 400) zurückgeführt wird, wobei das Wasser bevorzugt aus dem Verfahren ausgeschleust wird.
